# EUROPEAN PATENT APPLICATION

(11) **EP 0 792 621 A1**
(43) Date of publication of application: **03.09.1997**
(21) Application number: 97300681.0
(22) Date of filing: 04.02.1997
(51) Int. Cl.: A61B 17/04

(54) **Cannulated clamp**

(30) Priority: 29.02.1996 US 608681
(71) Applicant: Uwaydah, Munir, Dr., New York, New York 10023 (US)
(72) Inventor: Uwaydah, Munir, Dr., New York, New York 10023 (US)
(74) Representative: Charlton, Peter John

(57) **Abstract**

The application describes a clamping device, comprising:
(a) a guiding member with a guiding channel therethrough, wherein said guiding channel has an exit port; and
(b) a receiving member operatively connected to said guiding member, wherein said receiving member has a receiving device in alignment with said exit port of said guiding channel so that a needle can be guided out of said exit port of said guiding channel; and into said receiving device of said receiving member.

## Description

The present invention generally relates to a device and method for assisting a surgical procedure. Specifically, this invention relates to a type of clamp which allows a surgeon to capture tissue or bone that is difficult to reach and suture that captured material. More particularly, the method and device herein disclosed, allows a surgeon to easily and accurately guide a needle through tissue or bone, that would otherwise be difficult to suture.

Accurately guiding needles is always of prime concern to surgeons. Presently, many surgical procedures require guiding needles into and through areas that are difficult to reach.

When suturing is required in regions of the body where access is difficult or where placement of the suture is critical, a wide variety of clamps are available for the surgeon's use. Typically, the surgeon endeavors to reach into an area and grab a piece of tissue which may be difficult to capture. While this tissue is being held, the surgeon must accurately place a suture in a particular area of the tissue which is, itself, difficult to reach. This is often a tedious and difficult maneuver to accomplish, particularly in close critical areas. Further, the surgeon may have to attach one difficult to reach tissue with an even more difficult to reach point of fixation, when, for example, repairing torn connective tissue in the shoulder. This becomes a highly difficult operation to complete with full success. Often, a surgeon will settle on a point of attachment that is less than optimal because the desired placement of the suture is too inaccessible. This occurs with increasing frequency in areas which are difficult to access.

There are also many guiding devices well known in the surgical field for accurately placing devices in the body. Such guiding devices or cannulae guide other devices such as catheters to specific body locales. None of these devices are for grasping and holding tissue and for accurately guiding a needle therethrough.

There is therefore a great need in the art for a device which can securely grasp and hold material and which can allow a surgeon to accurately guide a needle therethrough. Accordingly, there is now provided with this invention an improved surgical clamp for effectively overcoming the aforementioned difficulties and long-standing problems inherent in general surgery, but especially in surgery involving regions of the body which are difficult to reach. These problems have been solved in a simple, convenient, and highly effective way by which to hold and secure tissue and accurately guide a needle through this tissue for making a suture. More particularly, a surgical clamp is provided which both grasps tissue or bone and which guides a needle therethrough. Additional objects of the present invention will become apparent from the following description.

According to one aspect of the invention, a clamping device is disclosed. The clamping device comprises a guiding member with a guiding channel therethrough. The guiding channel has an exit port. The device also has a receiving member operatively connected to the guiding member. The receiving member has a receiving device in alignment with the exit port of the guiding channel so that a needle can be guided through the guiding channel, out of the exit port of the guiding channel, and into the receiving device of the receiving member.

According to another aspect of the invention, a clamp is disclosed which comprises a first member with a channel therethrough. The channel has an entrance port and an exit port. The clamp also has a second member with a channel therethrough and this channel also has an entrance port and an exit port. When the members are secured to one another, the exit port of the channel of the first member is aligned with the entrance port of the channel of the second member. This is so that a needle can be inserted into the entrance port of the first member and guided through the channel of the first member, the exit port of the first member, the entrance port of the second member, the channel of the second member and out the exit port of the second member.

According to another aspect of the invention, a method of suturing is disclosed which comprises grasping a body part by a grasping means having at least two legs. A needle is inserted into and through a channel in one of the legs and the needle is passed through the body part and into and out of a channel in the other leg.

A further aspect of the invention includes a method for suturing comprising grasping a first body part with a first grasping means. The grasping means has a channel therein and the channel has an entrance and exit port. The method also comprises grasping a second body part with a second grasping means. The second grasping means has a channel therein and the channel has an entrance and an exit port. A needle is then passed through the channel of the first grasping means through the first and second body parts and into the channel of the second grasping means.

As will be appreciated by those persons skilled in the art, a major advantage provided by the present invention is to assist a surgeon performing a difficult surgical procedure. It is therefore an object of the present invention to provide a surgical device for assisting a surgeon in accurately placing sutures. It is another object to provide a surgeon with a device which will grasp and hold the tissue being sutured during the suturing process.

The method and apparatus of the present invention will be better understood by reference to the following detailed discussion of specific embodiments and the attached figures which illustrate and exemplify such embodiments.

A specific embodiment of the present invention will be described with reference to the following drawings, wherein:

Figure 1 is an orthogonal view of one embodiment of the present invention.

Figure 2 is an orthogonal view of a second embodiment of the present invention.

Figure 3 is an orthogonal view of a third embodiment of the present invention.

Figure 4 is an orthogonal view of a fourth embodiment of the present invention.

Figure 5 is an orthogonal view of a fifth embodiment of the present invention.

Figure 6 is an orthogonal view of a sixth embodiment of the present invention.

Figure 7 is an orthogonal view of a seventh embodiment of the present invention.

Figure 8 is an orthogonal view of a eighth embodiment of the present invention.

The following preferred embodiment as exemplified by the drawings is illustrative of the invention and is not intended to limit the invention as encompassed by the claims of this application. An apparatus and method for clamping and suturing is disclosed herein.

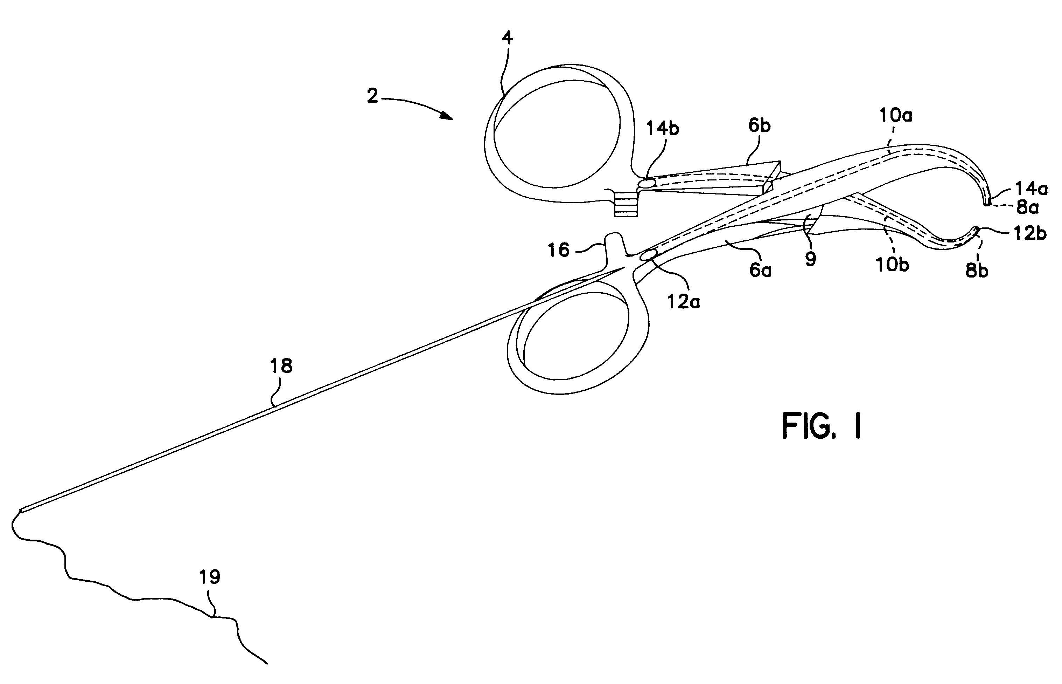

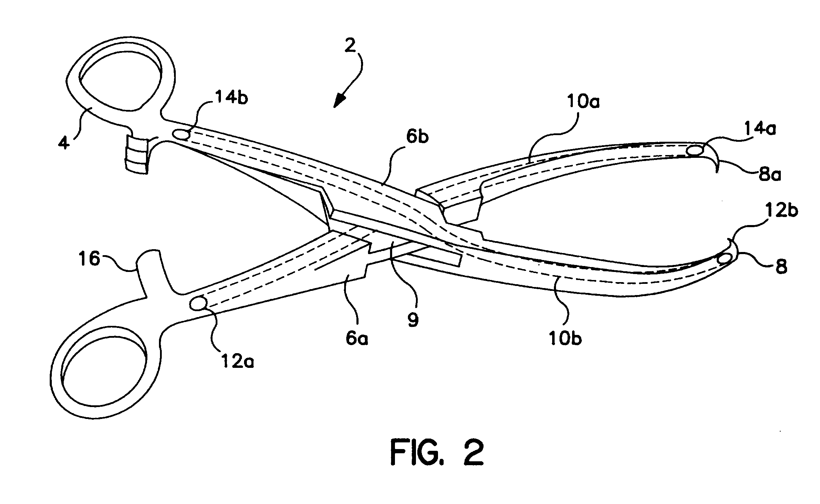

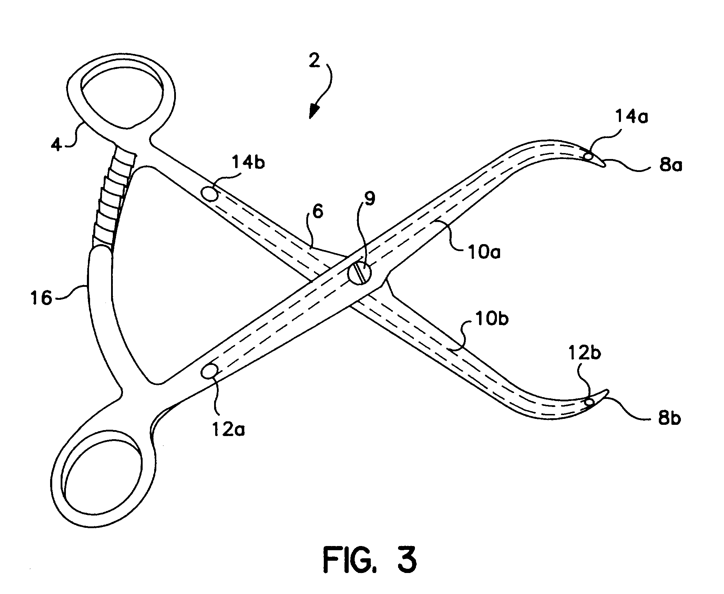

The apparatus, as illustrated generally in Figures 1 through 3, includes a clamping device or forceps (2). The clamping device typically has hand grips (4). The hand grips are used by the surgeon to guide and manipulate the clamping device in order to grasp the sought tissue. Such a clamping device typically has clamping arms (6a) and (6b) which extend from the hand grips and terminate at a clamping surface (8a) and (8b) respectively. One of the clamping arms acts as a guiding member (6a) and one of the clamping arms acts as a receiving member (8b).

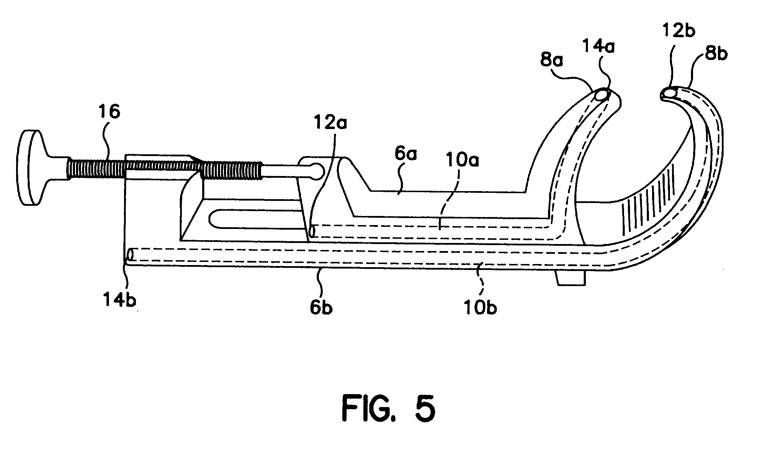

Often, as shown in Figures 14, the clamping arms pivot about one another in a scissor-like fashion about a pivot point (9). However, other types of clamping devices may also be used. For example, clamps exists which are known in the art as a Loman clamp. These securely hold tissue during surgical procedures and operate not using a scissor-type motion. Although the embodiments described herein with particularity address those clamps which operate in a scissor-like fashion, the invention is intended to be used in all types of grasping or clamping devices, as shown, for example, in Figure 5.

A channel or cannula (10a) and (10b) is formed within the clamping arms. Each channel has an entrance port (12a) and (12b) and an exit port (14a) and (14b) at opposite ends of each channel. The exit port of one channel is designed and formed to align with the entrance port of the other channel when the clamping surfaces (8a) and (8b) are brought together. The cannulae are formed to have a diameter suitable to pass a flexible needle therethrough. Typically, the diameter of the channel would be approximately 1mm. Of course, depending upon the size of both the needle used and the clamping arms themselves, this dimension may be somewhat greater or smaller than 1 mm.

Optionally, the clamping device may have a securing or locking member (16) which extends from one clamping arm to the other and thereby temporarily retains the clamping surfaces together. This securing member typically is formed as a ratchet and pawl mechanism or in a sloped tooth arrangement. Of course, any device which temporarily locks the arms together in order to keep the clamping surfaces temporarily together may be suitable.

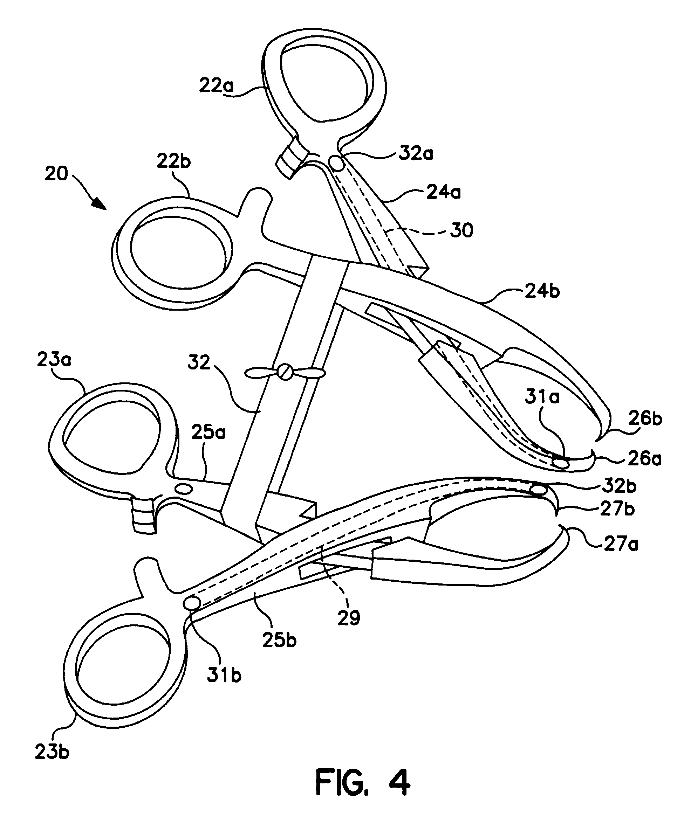

An alternative clamping and needle guiding apparatus is shown in Figure 4. The apparatus includes a clamping device (20). The clamping device generally has hand grips (22a, 22b, 23a, and 23b). The hand grips are used by the surgeon to guide and manipulate the clamping device in order to grasp the sought tissue. The clamping device (20) depicted in

Figure 4 has two sets of clamping arms (24a, 24b, 25a, and 25b) which extend from their respective handgrips and terminate as two sets of clamping surfaces (26a, 26b, 27a, and 27b). One set of clamping surfaces (for example, 26a and 26b) is for grasping and holding one body part and the other set of clamping surfaces (for example, 27a and 27b) are for grasping and holding a second body part.

Although both sets of clamping arms are shown to be pivotable in a scissor-like motion, one or both sets may also be a type of clamp which operate differently.

A channel or cannula (29 and 30) is formed in at least one of the clamping arms of each set of clamps. Each channel has an entrance port (31a and 31b) and an exit port (32a and 32b) at opposite ends of each channel. The exit port of one channel is designed and formed to align with the entrance port of the other channel when the two clamping surfaces are brought together.

Optionally, a securing means may be used to temporarily hold the clamping surface of one clamp with the clamping surface of the other clamp. This securing means may be any of a wide variety of means. Typically, one similar to a thumbscrew may be used, but of course, many forms of securing means are known to one skilled in the art, such as a cam-type locking device.

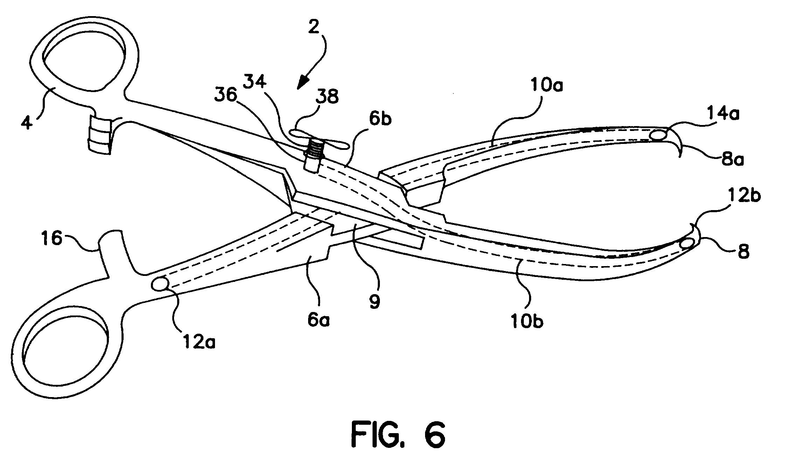

An alternative embodiment of the present invention is illustrated in Figure 6. One of the clamping arms acts as a guiding member (6a). The other of the clamping arms acts as a receiving member (6b). The receiving member has a receiving channel (10b) formed therein. The channel in the receiving member has an entrance port (12b) at its clamping surface end (8b). This receiving member channel does not have an exit port. Instead, a locking member (34) is positioned on the receiving member for locking a needle within the receiving channel. The locking member shown is positioned in a threaded channel (36). The threaded channel intersects with the receiving channel. A thumbscrew (38) is placed on the exposed end of the locking member. Although this locking member works similar to conventional lock nut design in the threaded channel, other kinds of locking devices may also be employed to secure a needle in the receiving channel.

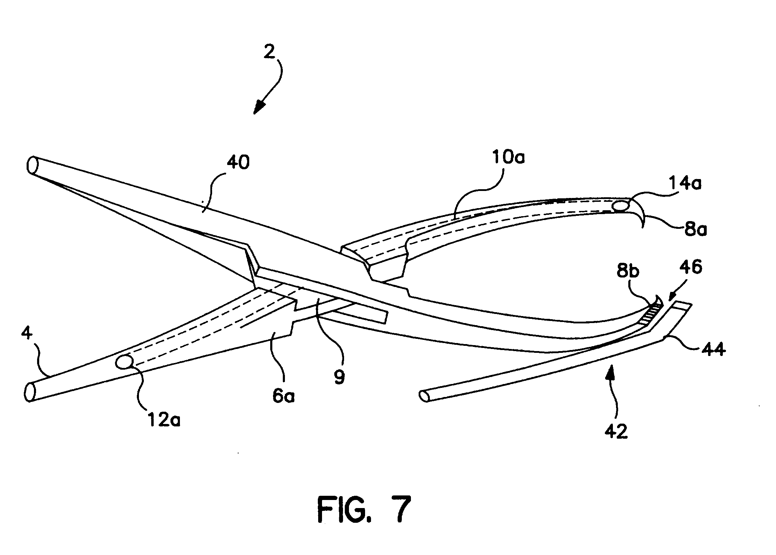

In an alternate embodiment illustrated in Figure 7, the receiving member 40 does not have a channel for capturing a needle. Instead, a receiving device (42) is positioned on the end of the receiving member in alignment with the exit port (14a) of the guiding channel (10a) of the guiding member (6a). The receiving device (42) depicted herein, is preferentially a clamping device, having at least one articulated member (44) for forming a pinching jaw (46) with the clamping surface (8b) of the receiving member.

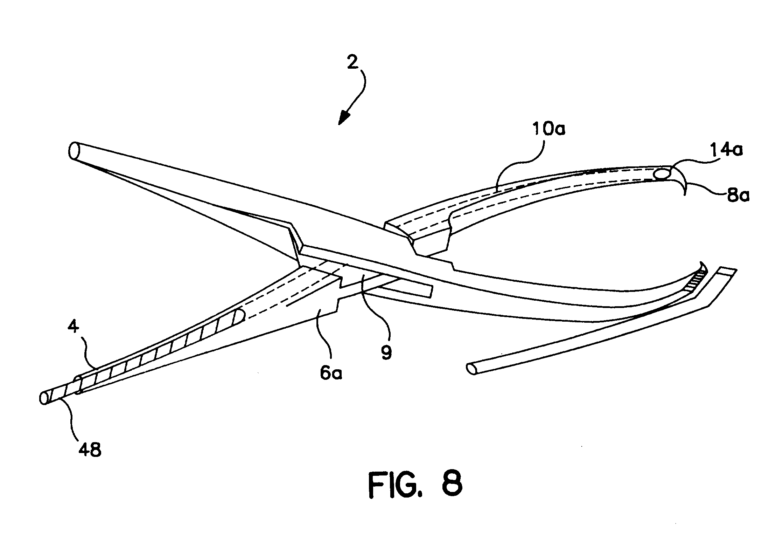

Figure 8 shows a further embodiment of the invention. In this embodiment, the guiding member (6a) has a guiding channel (10a) having an exit port (14a) at its clamping surface (8a) but it does not have an entrance port. Instead, a pushing device or plunger (48) is positioned at the distal end of the guiding channel (10a) from the exit port (14a). The plunger (48) is adapted to slide within the guiding channel in the guiding member thereby advancing a needle placed within.

Although specific examples of guiding members have been shown with specific examples of receiving members, it is to be understood, and it is fully contemplated by the inventor that any of the guiding members and their equivalents can be combined with any of the receiving members and their equivalents.

In practice, the disclosed device is intended to be used as follows. Using the handgrip, the surgeon will direct the device into the body cavity and secure the sought tissue. The clamping surfaces are then maneuvered to the precise site that the surgeon desires to affix a suture. The arms of the device are then held together, either by hand or by using a locking means. With the tissue held by the clamping surface at the precise suture point, a pre-threaded, long, yet flexible, surgical needle, similar to the one used for meniscal repair, is inserted into the entrance port of a channel of one of the arms of the device.

Once the needle is inserted into the entrance port of a channel, the channel guides the needle through the arm of the device as it is advanced through the channel by the surgeon. When the needle exits the exit port of the channel, because the tissue is being firmly held by the clamping surfaces and because the exit port of this channel is aligned with the entrance port of the other channel, the needle goes through the tissue precisely where the surgeon desires the needle to pass. The needle passes through the tissue and enters the entrance port of the channel in the other arm of the device. The surgeon continues to advance the needle through the channels until the entire length of the needle exits the exit port of the other arm.

Of course, the needle may not necessarily pass out of a receiving channel in the receiving member, it may also be secured within the channel by a locking device as shown in Figure 6. In fact, if the receiving member does not have a channel at all, as illustrated in Figures 7 and 8, then the needle is received and secured by a clamping or receiving device.

At this stage, the needle is separated from its attached thread and the clamp is guided off of both ends of the thread which has been entirely threaded through the tissue and the device thereby leaving the thread through the tissue. The suture has thus been placed precisely where the surgeon has desired. This operation can take place in a body area which has heretofore been unable to be accurately reached.

As will be appreciated by one skilled in the art, the needle should be of a strong yet flexible material. The needle must be able to flex at the place where it passes out of the exit port of one channel and into the entrance port of the other channel. Further, it must be long enough to extend all the way from the entrance port of one channel (for example, 12a) to the exit port of the other channel (for example, 14b). This is so the surgeon can continue to push the needle.

Other types of pushing may be used. For example, a plunger (48) as shown in Figure 8 may be used to advance a needle through the guiding channel if the needle is not long enough to span the entire transit from the entrance port of the first member to the exit port of the second member, or for other practical reasons.

The advantages of such a procedure, as described above, will be appreciated to those skilled in the art. For example, in an operation requiring a deep suture where the surgical incision is small, as in an anterior approach through a deltopectoral incision for Bankart lesion repair and anterior/inferior capsular shifts, suture placement is difficult. This is because the deep hole in which the operation takes place is very restrictive of the normal arc of motion that is required for suture placement using conventional techniques. Accurate placement of a clamp on the desired tissue, on the other hand, requires little, if any, arc of motion. By using the cannulated clamp as described, the suture can be accurately placed with a minimal arc of motion.

Although the particular embodiments shown and described above will prove to be useful in many applications in the surgical arts to which the present invention pertains, further modifications of the present invention herein disclosed will occur to persons skilled in the art. For example, model making also often requires placement of stitches in restrictive spaces. All such modifications are deemed to be within the scope and spirit of the present invention as defmed by the appended claims.

## Claims

1. A clamping device, comprising:
(a) a guiding member with a guiding channel therethrough, wherein said guiding channel has an exit port; and
(b) a receiving member operatively connected to said guiding member, wherein said receiving member has a receiving device in alignment with said exit port of said guiding channel so that a needle can be guided out of said exit port of said guiding channel; and into said receiving device of said receiving member.

2. The device of Claim 1, wherein said guiding member further comprises a pushing element adapted for sliding within said guiding channel.

3. The device of Claim 1, wherein said guiding channel has an entrance port distal from said exit port.

4. The device of Claim 1, wherein said receiving device comprises a gripper means.

5. The device of Claim 1, wherein said receiving device comprises a receiving channel with an entrance port.

6. The device of Claim 5, wherein said receiving member further comprises a locking member intersecting said receiving channel for locking a needle therein.

7. A clamping device, comprising:
(a) a first member with a channel therethrough, wherein said channel has an exit port; and
(b) a second member operatively connected to said first member, wherein said second member has a channel therethrough, wherein said channel in said second member has an entrance port in alignment with said exit port of said channel of said first member and an exit port, so that a needle can be inserted into said entrance port, guided through said channel of said first member, out of said exit port of said first member, and into said entrance port of said second member.

8. The device of Claim 7, wherein said connection between said first and second members is detachable.

9. The device of Claim 8, further comprising a clamping means for detachably securing said first member with said second member, wherein when said members are secured to one another said exit port of said first member is aligned with said entrance port of said second member.
